# EUROPEAN PATENT APPLICATION

(11) **EP 4 732 811 A2**
(43) Date of publication of application: **29.04.2026**
(21) Application number: 25211001.0
(22) Date of filing: 24.10.2025
(51) Int. Cl.: A61F 2/30, A61B 17/16, A61F 2/46, A61F 2/38

(54) **MODULAR AUGMENT FOR LONG BONE**

(30) Priority: 24.10.2024 US 202463711199 P
(71) Applicant: Howmedica Osteonics Corp., Mahwah, NJ 07430 (US)
(72) Inventor: PASCALE, Kenneth, HOBOKEN, 07030 (US); CHOUBEY, Sarah, GREEN BROOK, 08812 (US)
(74) Representative: Regimbeau

(57) **Abstract**

A modular implant (100) is provided and includes a first component (130) configured for receipt within a bone, the first component having a frustoconical shape and a lumen therethrough. Modular implant includes a second component (120) removably engaged to the first component such that a portion of the second component is received within the lumen of the first component. Further, modular implant includes a third component (110) removably engaged to the second component such that an inner surface (116) of the third component is engaged to an outer surface (123) of the second component, an outer surface (112) of the third component being configured for engagement with a resected portion of the bone.

## Description

### CROSS-REFERENCE TO RELATED APPLICATIONS

This application claims the benefit of the filing date of United States Provisional Patent Application No. 63/711,199 filed October 24, 2024, the disclosure of which is hereby incorporated by reference herein in its entirety.

### BACKGROUND

Total knee arthroplasty (TKA), knee replacement surgery, and revision TKA are oftentimes challenging procedures depending on the severity of the condition being treated. The removal or revision of a primary total knee prosthesis may result in moderate to severe bone loss, as well as ligamentous structures being compromised. Such bone loss may vary greatly in size, shape, and location. Bone loss may also leave remaining bone with quality which ranges from adequately dense and appropriate for biologic fixation, to soft and osteopenic bone better suited for cement fixation. Bone loss may be generally planar across a portion of a patient's tibial plateau, and at other times bone loss may span the entire width of the tibia. Further, bone loss may manifest as a cavitary defect, such as a truncated void into the metaphyseal region of the tibia. The location of the cavitary defect may be proximate to or more distant from the intramedullary ("IM") canal. Cavitary defects that are more distant from the IM canal may result in fixation challenges since the IM canal is almost always used for fixation purposes as it provides a stable anchoring means when used with an intramedullary stem. Furthermore, the bone loss may be a combination of both planar and cavitary defects.

The clinical success and longevity of a knee replacement depends primarily on the overall fixation and stability of the knee implant. Manufacturers of such implants offer various options to address the challenges listed above for knee replacement surgery. One such option is a cemented intramedullary stem offered to enhance fixation of the implant within the IM canal.

However, there are challenges associated with ensuring long-term fixation and stability of cemented stems. Currently available stems are cemented within the IM canal which prevents direct biological fixation of the stem with the host bone Moreover, enhancing biological fixation of the implant with augments and collars is difficult. Currently available implants require augments and collars to be assembled to stems prior to implantation within a bone of a patient. Further, when the IM canal is pre-filled with bone cement, the bone cement extrudes out of the IM canal along the prepared bone fixation surfaces as the stem is implanted into the IM canal, which cause the bone cement to entirely separate augment and/or collar from the bone. This movement of bone cement degrades any potential biological fixation between the augment, collar, or other components of the implant with the bone.

Therefore, implants and associated methods for enhanced biological fixation between an implant and a bone during knee arthroplasty are desirable.

### BRIEF SUMMARY

In a first example of a first aspect, the present disclosure relates to a modular implant. The modular implant includes a first component configured for receipt within a bone, the first component having a frustoconical shape and a lumen therethrough. Additionally, the modular implant includes a second component removably engaged to the first component such that a portion of the second component is received within the lumen of the first component. Further, the modular implant includes a third component removably engaged to the second component such that an inner surface of the third component is engaged to an outer surface of the second component, an outer surface of the third component being configured for engagement with a resected portion of the bone.

In a second example, the first example of the first aspect is further defined wherein portions of the first component and the third component that interface with the second component are made of materials different from the second component. In a third example, the second example of the second aspect is further defined wherein the second component is made of a polymeric material and at least a portion of the respective first and third components interfacing with the second component is made of a metallic material.

In a fourth example, the first example of the first aspect is further defined wherein the third component is ring-shaped and has a diameter greater than a maximum diameter of the first component. In a fifth example, the first example of the first aspect is further defined wherein the first component includes a first threaded surface, the second component includes a second threaded surface and a third threaded surface and the fourth component includes a fourth threaded surface, the first threaded surface being engaged with the second threaded surface and the third threaded surface being engaged with the fourth threaded surface. In a sixth example, the first example of the first aspect is further defined wherein when the second component is engaged to the first component, the second component extends only partially into the lumen of the first component.

In a seventh example, the first example of the first aspect is further defined wherein the first component is made of a solid material and a porous material, wherein the porous material is disposed over an outer surface of the solid material. In an eight example, the seventh example of the first aspect is further defined wherein the porous material is configured to enhance biological fixation with the bone. In a ninth example, the first example of the first aspect is further defined wherein the third component is made of a solid material and a porous material, wherein the porous material is disposed over an outer surface of the solid material. In a tenth example, the ninth example of the first aspect is further defined wherein the porous material is configured to enhance biological fixation with the bone. In an eleventh example, the first example of the first aspect is further defined wherein a tab extends from a proximal surface of the second component, the tab being configured to engage a distal surface of the third component and partially form a gap between the proximal surface of the second component and the distal surface of the third component. In a twelfth example, the first example of the first aspect is further defined wherein a notch is formed within an outer surface of the third component, the notch being configured to be engageable by an instrument.

In a thirteenth example, a system comprises a modular implant of the first example of the first aspect and further comprises a stem disposed through the lumen of the first component and within the bone; and bone cement configured to fixate the modular implant and stem to the bone.

In a first example of a second aspect, the present disclosure relates to a modular implant of another embodiment. The modular implant includes a first component having a lumen extending therethrough and being configured for receipt within a bone, the first component including a porous portion and a solid portion and having a frustoconical shape. Additionally, the modular implant includes a second component removably engaged to the first component such that a portion of the second component is received within the lumen of the first component, the second component comprising a porous portion and solid portion. The porous portions of the first and second components are configured for engagement with a resected portion of bone, wherein at least a part of the solid portions are configured to engage bone cement.

In a second example of the second aspect, the first example is further defined wherein the second component has a maximum outer dimension greater than a maximum outer dimension of the first component. In a third example of the second aspect, the first example is further defined wherein the first component tapers from a first end that is releasably engaged to the second component to a second end opposite the first end. In a fourth example of the second aspect, the first example is further defined wherein the lumen of the first component tapers over at least part of a distance from a first end of the first component to a second end of the first component opposite the first end. In a fifth example of the second aspect, the first example is further defined wherein a lumen of the second component includes at least one stepped surface. In a sixth example of the second aspect, the first example is further defined wherein the second component further comprises a first subcomponent and a second subcomponent releasably engaged to the first subcomponent.

In a first example of a third aspect, the present disclosure relates to a method. The method comprises the steps of positioning a frustoconical implant component into a cavity in a bone such that a leading surface of the frustoconical implant contacts a first bone surface inside the cavity; and engaging a collar component with the frustoconical implant by advancing a portion of the collar component into a lumen of the frustoconical implant until a leading surface of the collar component external to the frustoconical implant contacts a second bone surface of the bone, the second bone surface being outside of the cavity.

In a second example of the third aspect, the first example further comprises, prior to engaging the collar component with the frustoconical implant, engaging a connector component with the frustoconical implant by advancing a portion of the connector component into the lumen of the frustoconical implant. In a third example of the third aspect, the first example further comprising advancing a stem through the collar component and the frustoconical implant when the collar component is engaged to the frustoconical implant. In a fourth example of the third aspect, the third example further comprises, prior to the step of advancing the stem, introducing bone cement through the collar component and the frustoconical implant into the cavity. In a fifth example of the third aspect, the first example further comprises after the step of advancing the stem, introducing bone cement through the collar component and the frustoconical implant into the cavity.

### BRIEF DESCRIPTION OF THE DRAWINGS

A more complete appreciation of the subject matter of the present disclosure and the various advantages thereof may be realized by reference to the following detailed description which refers to the accompanying drawings, in which:
FIG. 1A is a perspective view of a modular diaphyseal augment according to an embodiment of the present disclosure;
FIG. 1B is an exploded view of the augment of FIG. 1A;
FIG. 1C is an exploded, cross-sectional view of the augment of FIG. 1A;
FIG. 2A is a side view of a modular diaphyseal augment according to an embodiment of the present disclosure;
FIG. 2B is a perspective view of the augment of FIG. 2A;
FIG. 2C is a top view and a bottom view of the augment of FIG. 2A;
FIG. 2D is an exploded view of the augment of FIG. 2A;
FIG. 2E is a cross-sectional view of the augment of FIG. 2A;
FIG. 2F are top, side and bottom views of a collar of the augment of FIG. 2A;
FIG. 2G are top, side and bottom views of a body of the augment of FIG. 2A;
FIG. 3A is a perspective view of a modular diaphyseal augment according to an embodiment of the present disclosure;
FIG. 3B is an exploded, cross-sectional view of the augment of FIG. 3A;
FIG. 3C is an assembled, cross-sectional view of the augment of FIG. 3A;
FIGS. 4A-4G show steps in a method of using a modular diaphyseal augment according to an embodiment of the present disclosure;
FIGS. 5A-5F show steps in another method of using a modular diaphyseal augment according to an embodiment of the present disclosure; and
FIGS. 6A-6G show steps in another method of using a modular diaphyseal augment according to an embodiment of the present disclosure.

### DETAILED DESCRIPTION

As used herein, the terms "about," "generally," and "substantially" are intended to mean that slight deviations from absolute are included within the scope of the term so modified. To aid the Patent Office and any readers of any patent issued on this application in interpreting the claims appended hereto, Applicant notes that it does not intend any of the appended claims or claim elements to invoke 35 U.S.C. § 112(f) unless the words "means for" or "step for" are explicitly used in the particular claim.

As used herein, when used in connection with the human body, the term "proximal" means closer to the heart, and the term "distal" means further from the heart. As used herein, the terms "substantially," "generally," "approximately," and "about" are intended to mean that slight deviations from absolute are included within the scope of the term so modified.

In one aspect, the present disclosure relates to an implant receivable within a bone. In some embodiments, an implant is adapted for placement in a long bone, such as a modular diaphyseal augment receivable within an intramedullary (IM) canal of a long bone. It should be appreciated that although the embodiments described herein and depicted in the Figures are applicable to a femur or tibia, the principles of the present disclosure may be applied to other long bones within a patient's body.

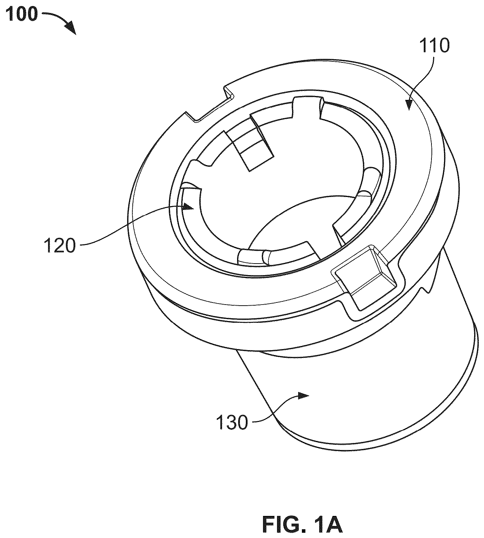

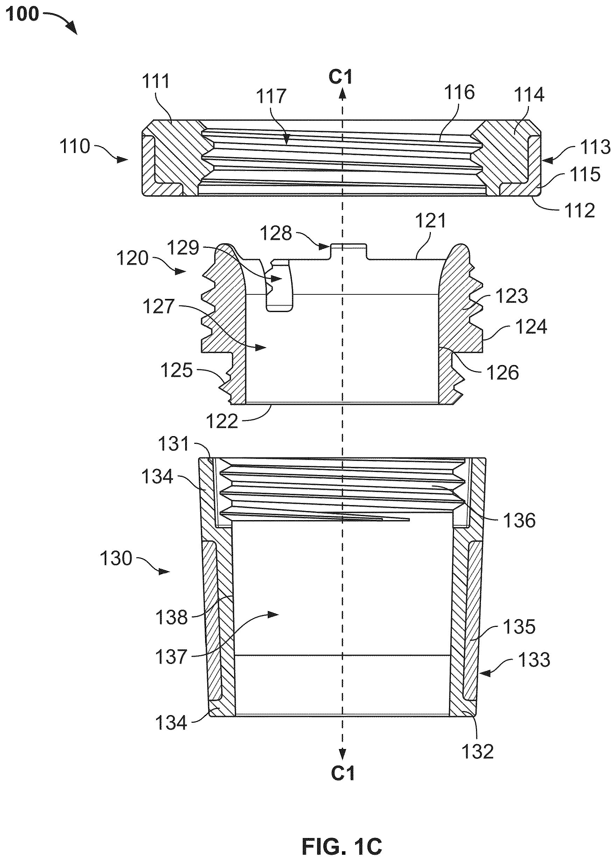

FIGS. 1A-1C illustrate a modular diaphyseal augment 100 according to one embodiment of the present disclosure. In one example, augment 100 is used in a total knee arthroplasty (TKA) or revision TKA procedure for securing a stem or adapter within the IM canal of a long bone, e.g., femur or tibia.

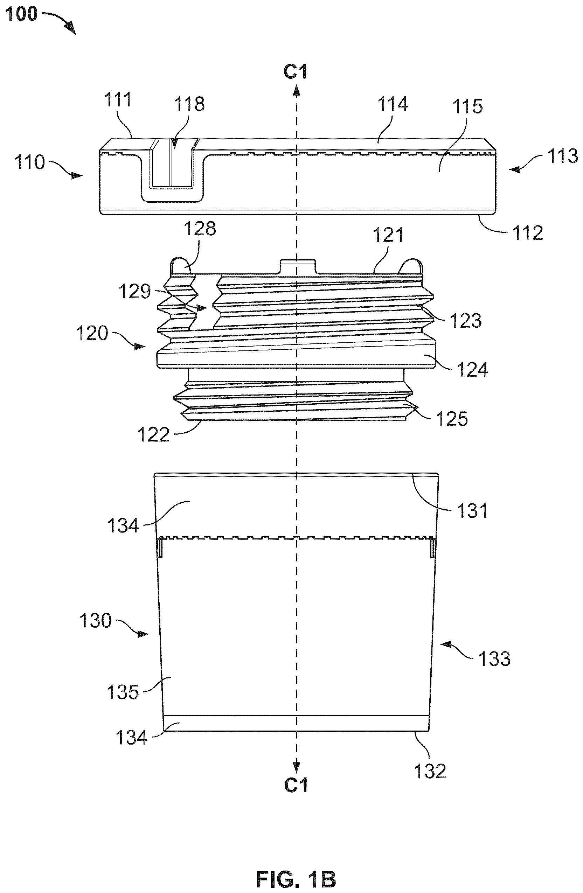

Augment 100 includes a collar 110, a locking connector 120, and a body 130. Collar 110 is positioned at a proximal end of an assembled augment 100. Collar 110 is circular in shape and includes an outer circumferential surface 113 extending in a depth direction from a first end 111 to a second end 112 opposite first end 111. Outer surface 113 includes a solid portion 114 adjacent first end 111 and a porous portion 115 adjacent second end 112. Solid portion 114 is comprised of a solid material, such as titanium alloy, while porous portion 115 is comprised of a porous structure for enhancing biological fixation and to promote bone ingrowth. Solid portion 114 defines an inner portion of collar 110 while porous portion 115 defines an outer portion or layer of collar 110 and partially defines outer surface 113. In the depicted embodiment, porous portion 115 extends along an outer surface of collar 110 from a depth adjacent to first end 111 to second end 112. Collar 110 further includes a central opening 117 extending longitudinally from first end 111 to second end 112 and is defined by a threaded inner surface 116. Within opening 117, threaded inner surface 116 extends from first end 111 to second end 112 and is configured to mate with a portion of connector 120, as discussed further below. As shown in FIG. 1B, outer surface 113 further includes a notch 118 disposed partially therein. Among other uses, notch 118 is configured to engaged by an instrument (e.g., introducer) to thread collar 110 onto first threaded outer surface 123 of locking connector 120 and seat collar 110 adjacent the bone.

Body 130 is positioned at a distal end of an assembled augment 100. Body 130 may be cylindrical or conical in shape and includes a circumferential outer surface 133 extending in a depth direction from a first end 131 to a second end 132 opposite first end 111. Outer surface 133 includes a first solid portion 134 adjacent first end 131 and a second solid portion 135 adjacent second end 132. Solid portion 134 is comprised of a solid material, such as titanium alloy, while porous portion 135 is comprised of a porous structure for enhancing biological fixation and to promote bone ingrowth. Body 130 further includes a central opening 137 extending longitudinally from first end 131 to second end 132. A portion of an inner surface that defines opening 137 and is adjacent to first end 131 is a threaded inner surface 136. Threaded inner surface 136 is configured to mate with a portion of connector 120, as discussed further below. Further, a portion of a smooth inner surface 138 that defines opening 137 and is adjacent second end 132 is a non-threaded inner surface and is configured to receive a stem and contact bone cement received be augment 100 when implanted, as discussed further below.

Connector 120 is positioned distal of collar 110 and proximal of body 130 when augment 100 is assembled. Connector 120 may be cylindrical or conical in shape and includes a first threaded outer surface 123 adjacent to a first end 121 and a second threaded outer surface 125 adjacent to a second end 122 opposite first end 121. Each threaded outer surface 123, 125 are separated by a seam 124 and are circumferentially disposed between first and second ends 121, 122. First threaded outer surface 123 of connector 120 is configured to mate with threaded inner surface 116 of collar 110, and second threaded outer surface 125 of connector 120 is configured to mate with threaded inner surface 136 of body 130. Connector 120 further includes a plurality of tabs 128 extending from first end 121 in a direction away from second end 122. Tabs 128 are configured to enhance the receipt of the stem receivable through augment 100. Additionally, tabs 128 maintain a gap between the stem and first end 111 of collar 110 to ensure that bone cement fills the gap and space between tabs 128, as discussed further below. First threaded surface 123 further includes a notch 129 partially disposed therethrough. Notch 129 is configured to be engaged by an instrument (e.g., introducer) to thread the second threaded outer surface 125 of connecter 120 into body 130 and seat the seam 124 of connector 120 adjacent to first end 131 of body 130. Additionally, in a fully assembled augment 100, a central longitudinal axis C1 extends through each of the respective openings 117, 127, 137 of collar 110, connector 120, and body 130, respectfully. Further, while augment 100 is shown with a threaded connection between collar 110 and body 130, in some examples, connector 120 may include one or more of a camlock (frictional lock), snap-fit (push or twist; spring-lock), splined dowel, compression lock, spring-loaded tabs, and ratchet mechanism.

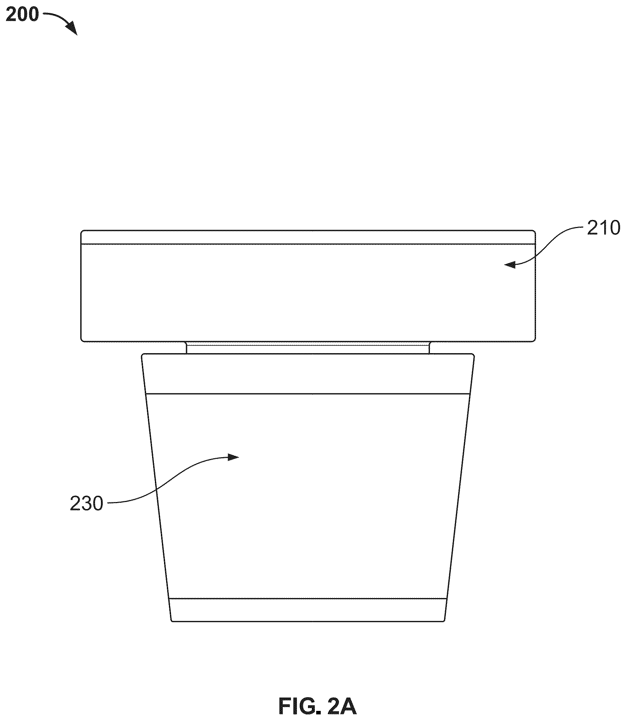

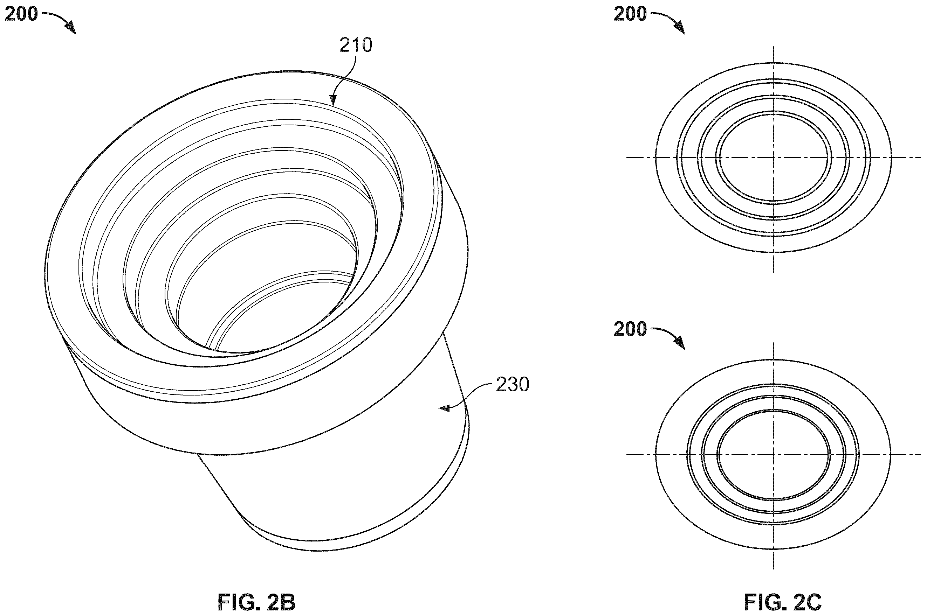

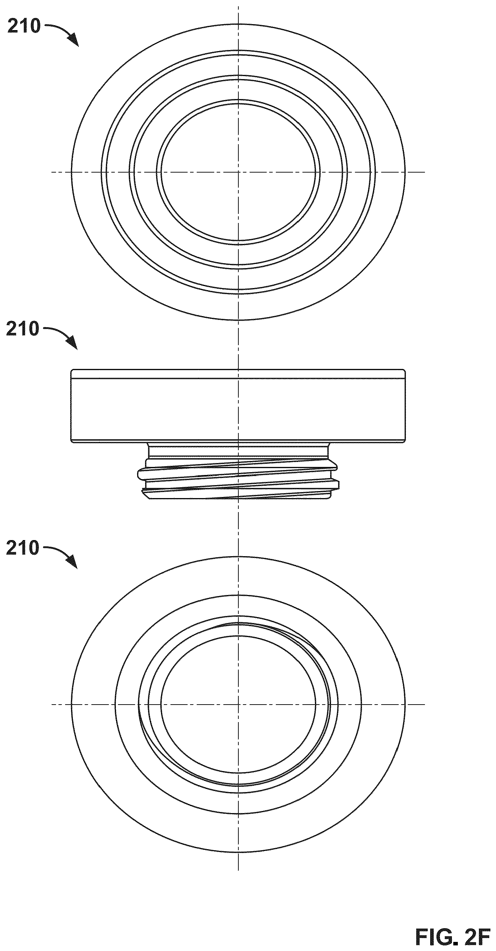

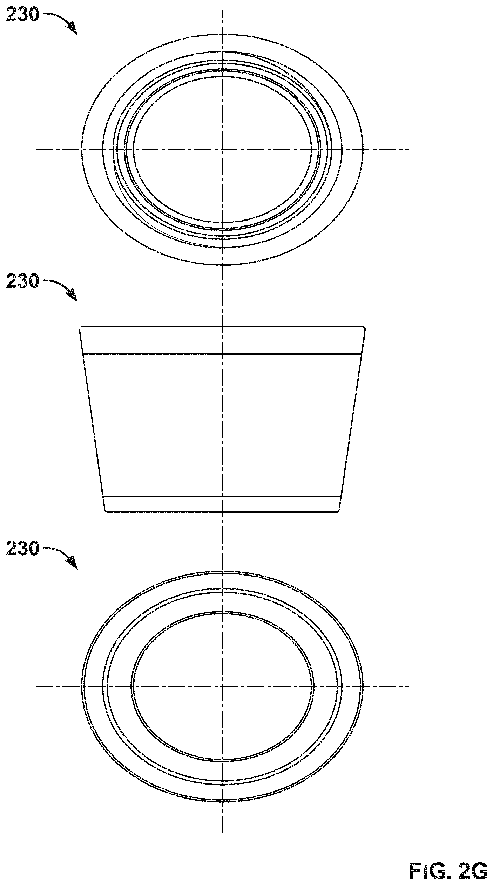

FIGS. 2A-2G illustrate a modular diaphyseal augment 200 according to another embodiment of the present disclosure. Augment 200 includes similar components to augment 100, but with reference numerals within the 200-series of numbers. For example, augment 200 includes a collar 210, connector 220, and body 230. However, in augment 200, collar 210 and connector 220 differ as described below.

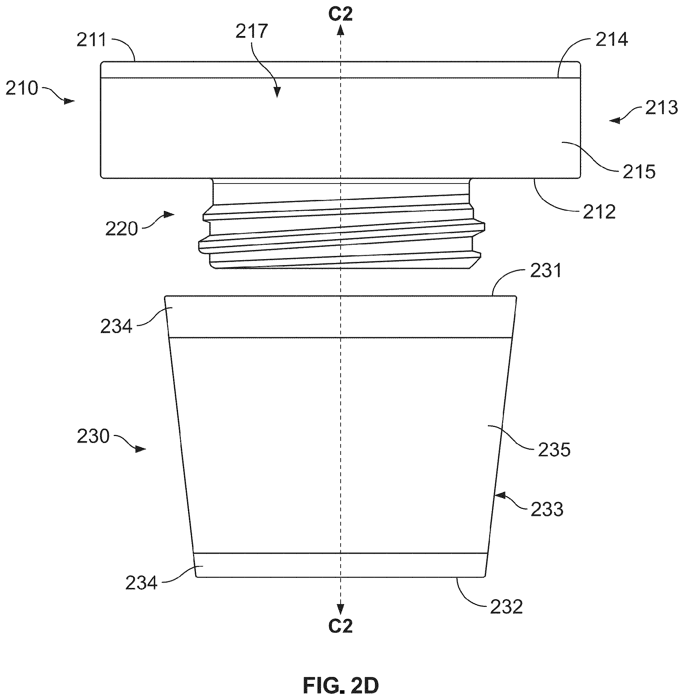

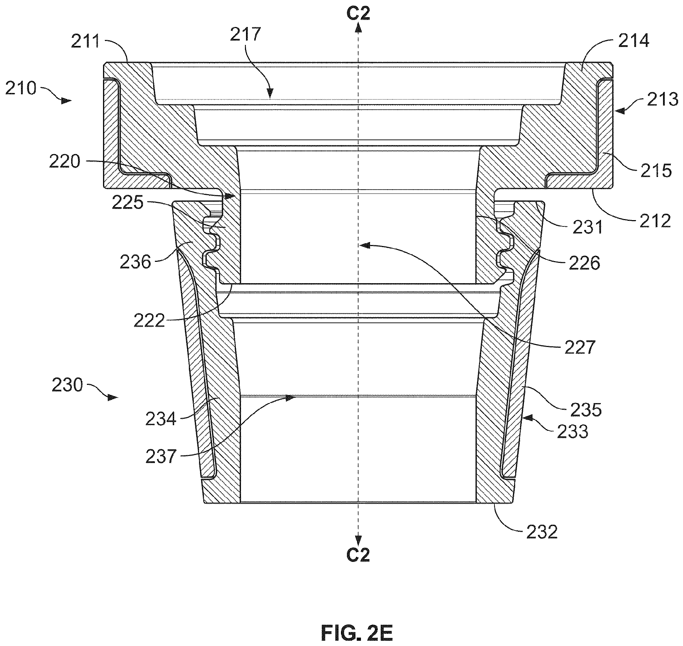

As shown in FIGS. 2D and 2E, connector 220 is integral with collar 210 and extends distally from second end 212 of collar 210. In some examples, connector 220 is monolithically formed with collar 210 and in other examples, such components are fixed to each other. Additionally, opening 217 of collar 210 is continuous and in fluid communication with opening 227 of connector 220 such that a central longitudinal axis C2 of augment 200 extends through the respective openings 217, 227, 237 of collar 210, connector 220, and body 230, respectively, when augment 200 is fully assembled. Therefore, unlike augment 100, connector 220 of augment 200 is not a separate component and directly connects collar 210 with body 230. Further, while augment 200 is shown with a threaded connection between threaded outer surface 225 of collar 210 and threaded inner surface 236 of body 230, in some examples, connector 220 may include a camlock (frictional lock), snap-fit (push or twist; spring-lock), splined dowel, compression lock, spring-loaded tabs, or ratchet mechanism.

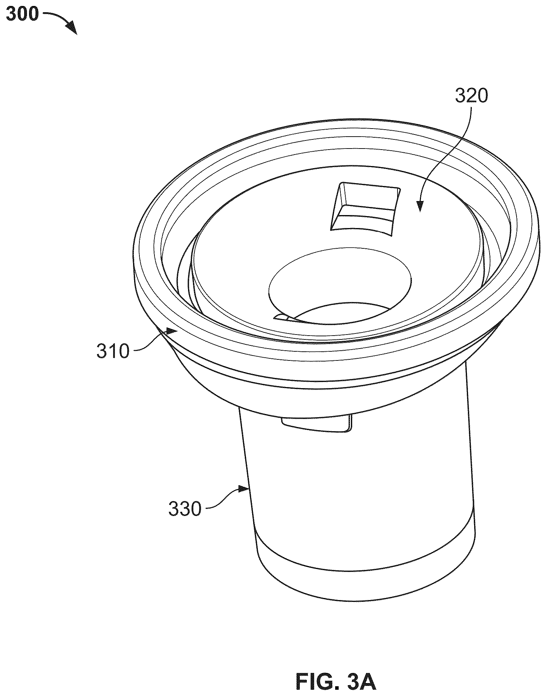

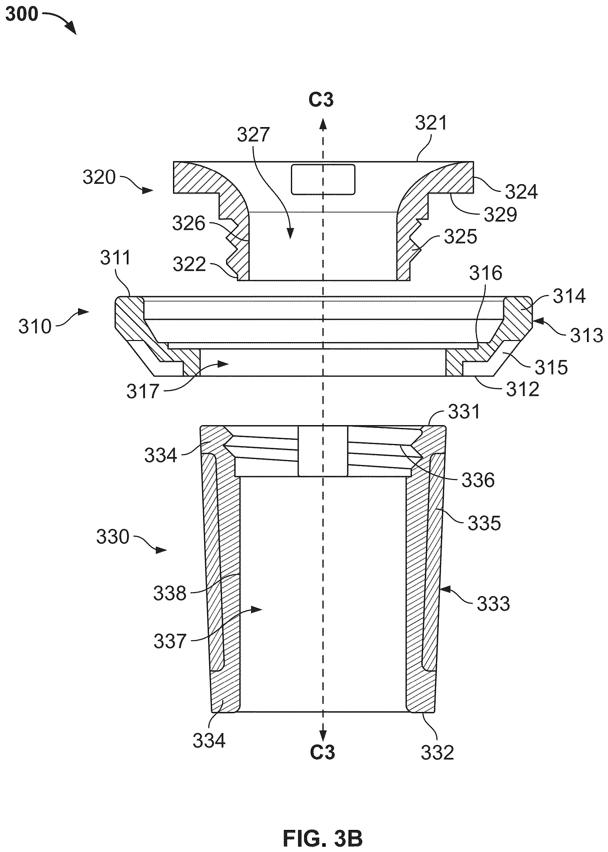

FIGS. 3A-3C illustrate a modular diaphyseal augment 300 according to another embodiment of the present disclosure. Augment 300 includes similar components to augments 100, 200, but with reference numerals within the 300-series of numbers. For example, augment 300 includes a collar 310, connector 320, and body 330. However, in augment 300, collar 310 and connector 320 differ as described below.

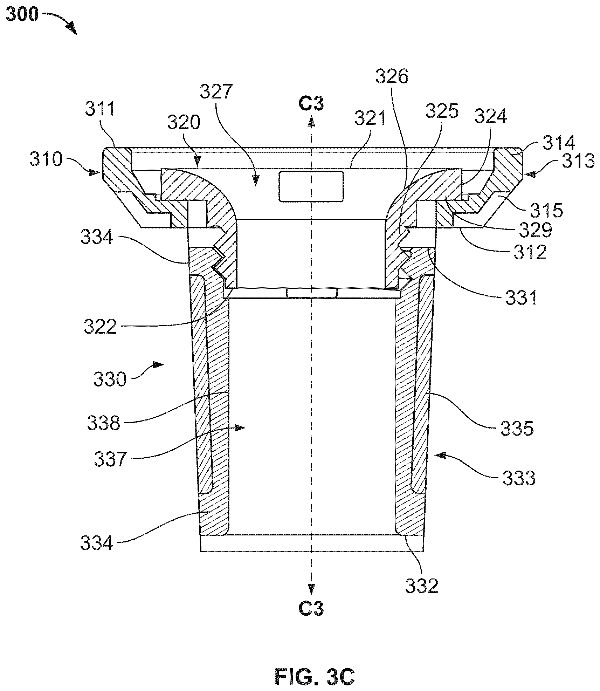

Connector 320 may be cylindrical or conical in shape and includes a threaded outer surface 325 adjacent to a second end 322 opposite a first end 321. Threaded outer surface 325 and first end 321 are separated by a seam 324 and is circumferentially disposed between first and second ends 321, 322. Seam 324 includes a step 329 adjacent threaded outer surface 325. Threaded outer surface 325 of connector 320 is configured to mate with threaded inner surface 336 of body 330, and step 329 of seam 324 is configured to contact a step 316 of collar 310, as shown in FIG. 3C. Therefore, when augment 300 is assembled, connector 320 secures collar 310 to both body 330 and/or the bone. Additionally, when augment 300 is assembled, as shown in FIG. 3C, a gap may be optionally formed between collar 310 and body 330 to receive bone cement. Further, while augment 300 is shown with a threaded connection between connector 320 and body 330, in some examples, connector 320 may include one or more of a camlock (frictional lock), snap-fit (push or twist; spring-lock), splined dowel, compression lock, spring-loaded tabs, and ratchet mechanism.

In another aspect, the present application relates to a kit including a combination of components including at least one component of augment 100, 200, 300. In some embodiments, a kit may include one or more components from augment 100, 200, 300. For example, a kit may include a plurality of collars 110, 210, 310 a plurality of connectors 220, 320 and a plurality of bodies 130, 230, 330. In other examples, a kit may include at least one collar, at least one connector and at least one body. In still further examples, a kit may include at least one collar and at least one body. In other embodiments, the kit may include a reamer and additional reaming attachments, such as an IM canal reaming attachment, a conical reaming attachment, and a planar reaming attachment. In even more embodiments, the kit may further include trialing components such as stem trials and collar trials.

Any combination of system components may also be included in a single package or in individual packages which may later be brought together to create a kit. When more than one package is used, any one package in such kit may include one or more of the components of the kit. It is also contemplated that a kit may include any combination of components along with one or more additional instruments used to place such securement devices in a patient. In other examples, the kits contemplated herein may be accompanied by an instruction manual on how to perform one or more of the methods of using the contents of the kit.

In another aspect, the present disclosure relates to a method for performing a surgical procedure for preparing a bone to receive an implant. In some embodiments, a method may be specifically directed to preparing a long bone to receive an implant during a TKA or revision TKA procedure. As explained elsewhere in the present application, it should be appreciated that the system and components thereof as contemplated by the present disclosure are not limited to use in TKA or revision TKA procedures. And, to the extent the methods below are described with respect to TKA and revision TKA procedures, such description is for purposes of illustration. It should be understood that the following operations do not have to be performed in the exact order described below. Instead, various steps may be handled in a different order or simultaneously. Steps may also be omitted or added unless otherwise stated herein.

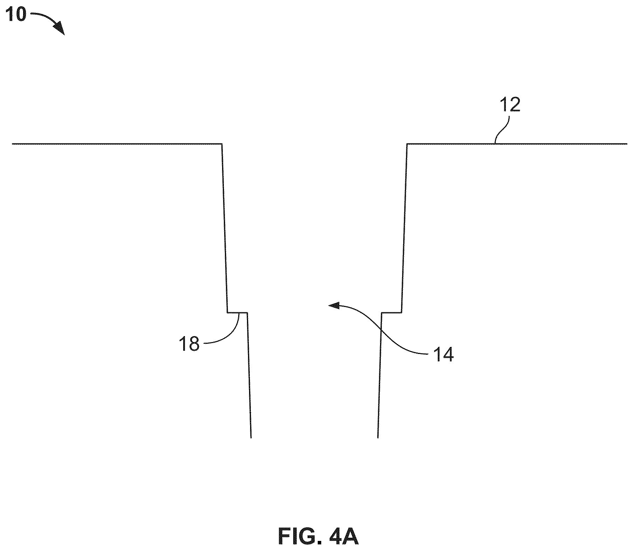

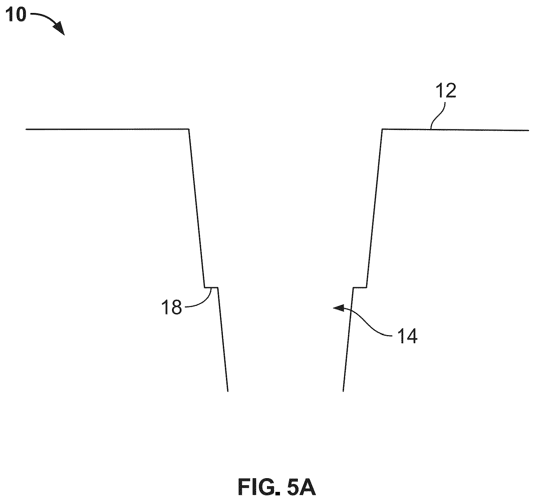

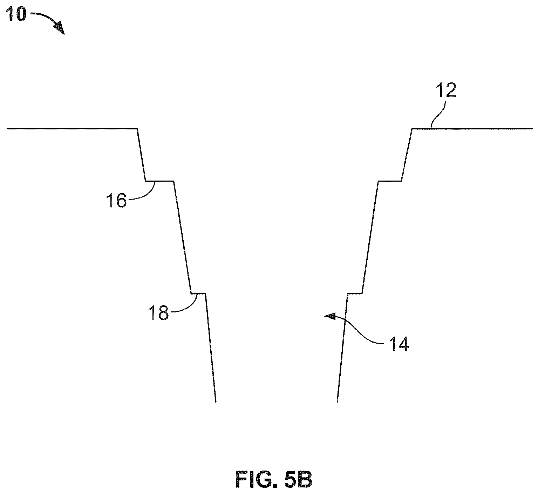

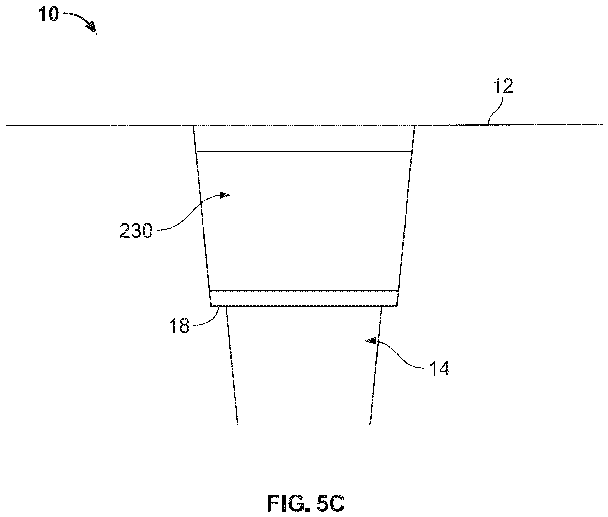

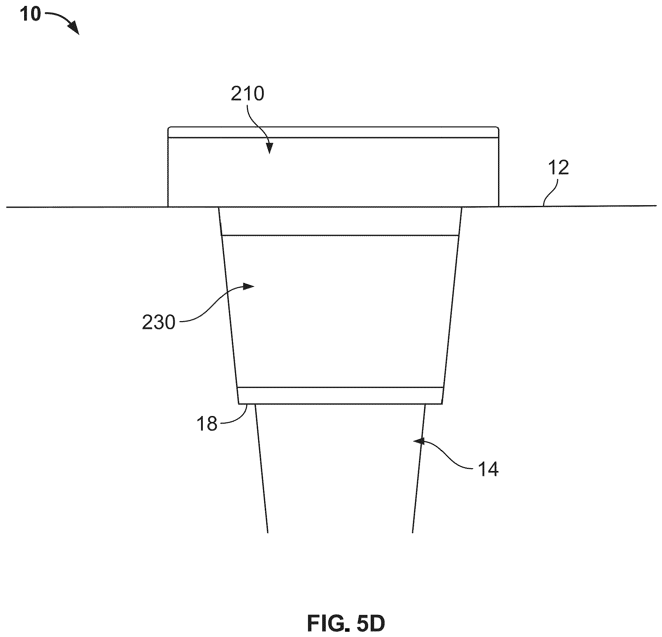

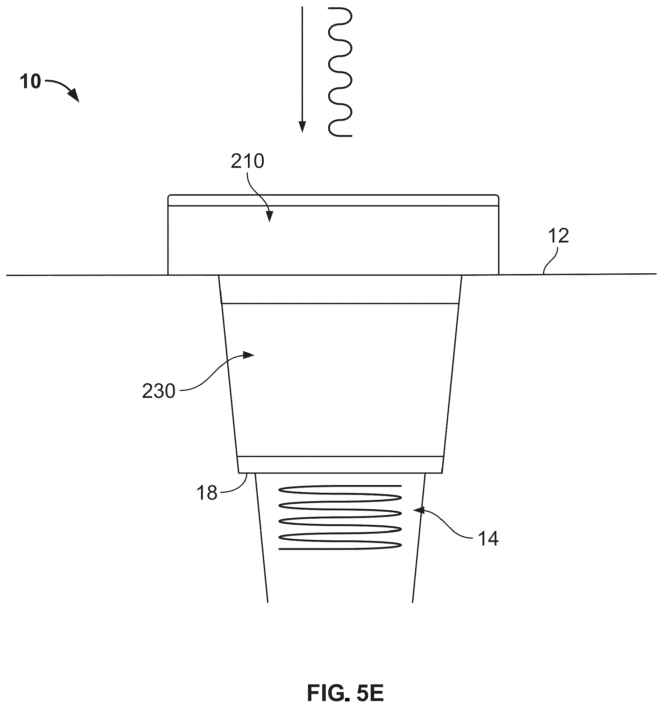

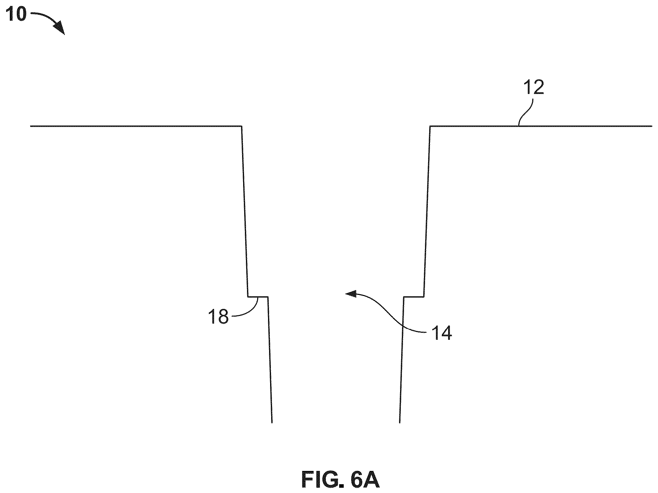

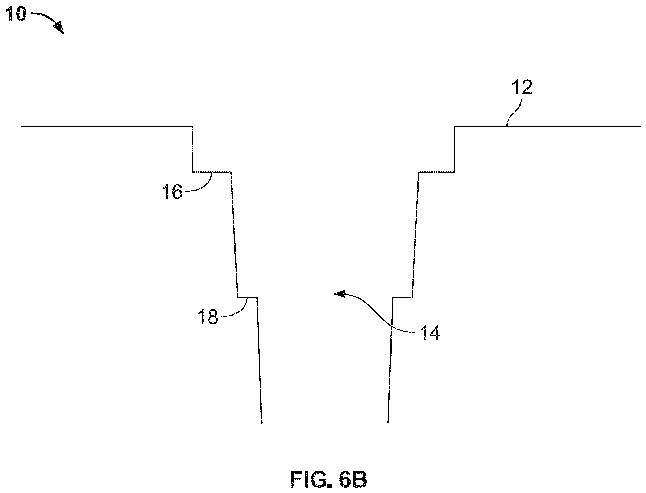

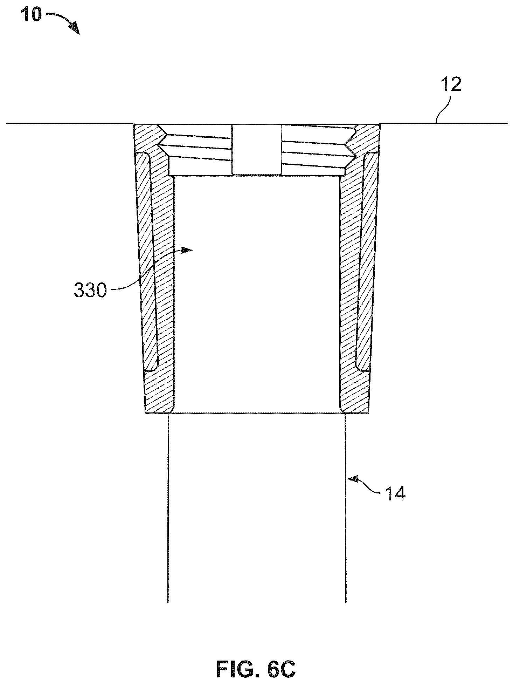

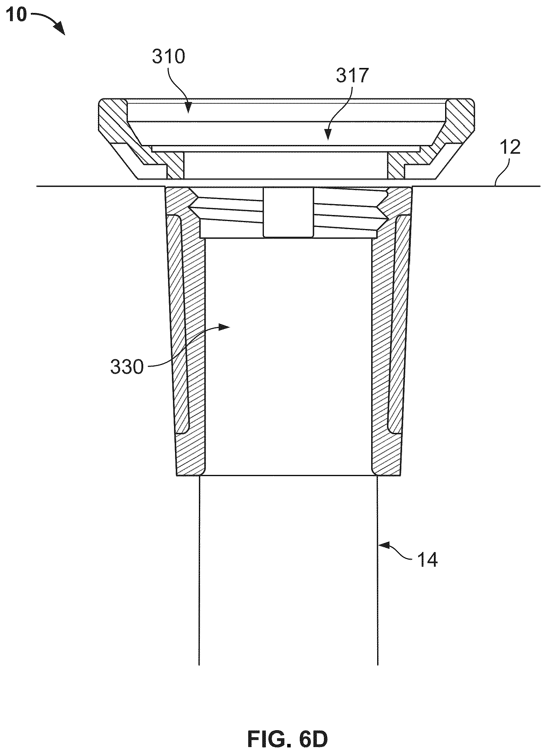

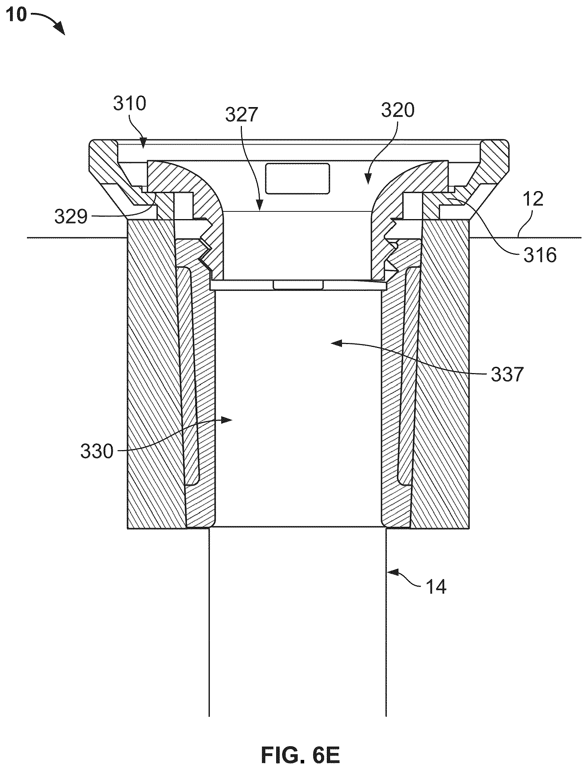

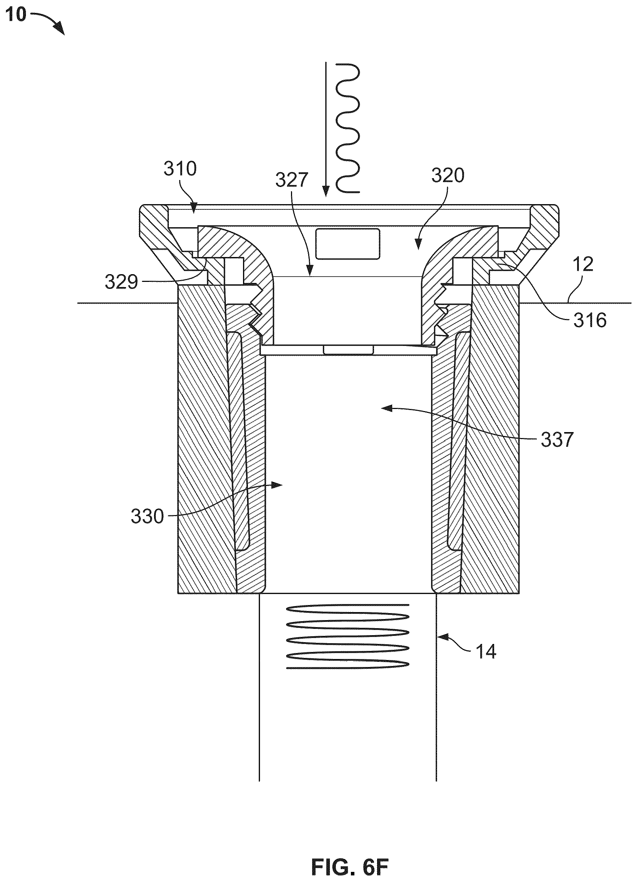

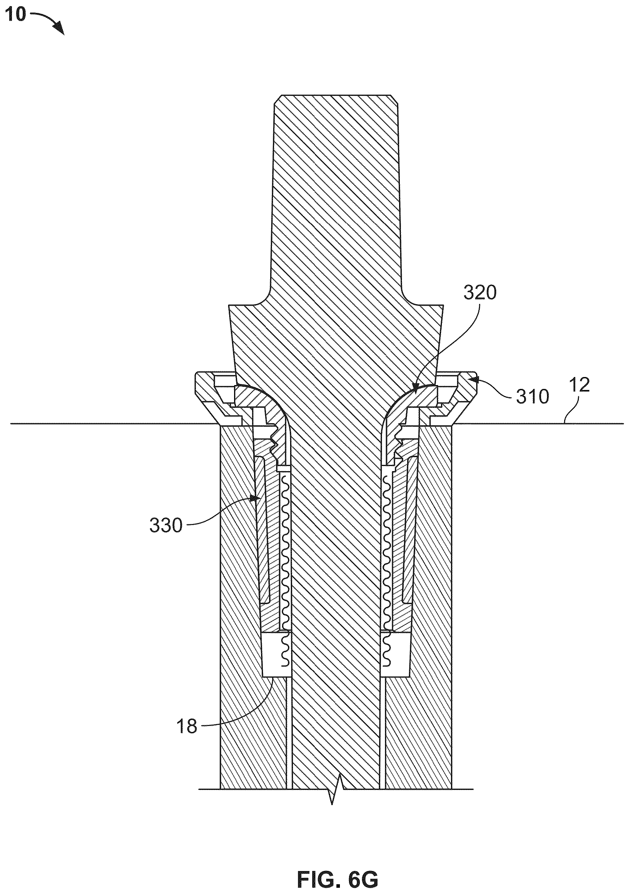

In one embodiment, a method for preparing a bone 10 for receiving an implant with augment 100 is illustrated in FIGS. 4A-4G. In a second embodiment, a method for preparing a bone 10 for receiving an implant with augment 200 is illustrated in FIGS. 5A-5F. In a third embodiment, a method for preparing a bone 10 for receiving an implant with augment 300 is illustrated in FIGS. 6A-6G. In all embodiments, the methods begin with a first step of performing an osteotomy or resection at a desired resection surface 12 of the bone 10, as shown in FIGS. 4A, 5A, and 6A. As previously mentioned, the portion of the bone 10 prepared for receiving the implant may be a distal femur, proximal femur, or proximal tibia, but may also be other long bones within the patient.

In a second step, the IM canal 14 of the of bone 10 is initially reamed by an IM canal reamer, as shown in FIGS. 4A, 5A, and 6A. In some examples, reaming includes use of IM canal reamers with progressively larger diameters until a desired opening size is formed. In a third step, a diameter of a stem to be implanted within the IM canal 14 is determined. The diameter of the stem is complementary to a diameter of the reamed IM canal 14, and the diameter of the stem is selected based upon a determined diameter of IM canal reamer used to ream the bone 10. In a fourth step, a conical reamer is assembled with a selected conical reamer component sized to complement the diameter of the chosen stem. With the selected conical reamer component, a conical cavity is formed in the IM canal 14 and the bone 10 is further reamed in preparation for receiving augment 100, 200, 300. Specifically, a distal recess step 18 is formed within the IM canal 14 using the conical reamer, such distal recess step 18 being configured for receiving a distal end of the respective augment 100, 200, 300.

In a fifth step, the portion of the canal reamed via the conical reamer is trialed to determine a size of the augment to be implanted. In some examples, the conical reamer may be used as the trial. Specifically, after reaming a portion of the IM canal 14 with the selected conical reamer component, the conical reamer component may optionally be detached from the conical reamer and positioned within the reamed portion of the bone 10. In this manner, while positioned within the bone 10, the conical reamer component may serve as a conical trial. Alternatively, a separate conical trial may be used to determine the size of the augment to be implanted. Next, a stem trial is inserted through the conical reamer component or other trial, as applicable.

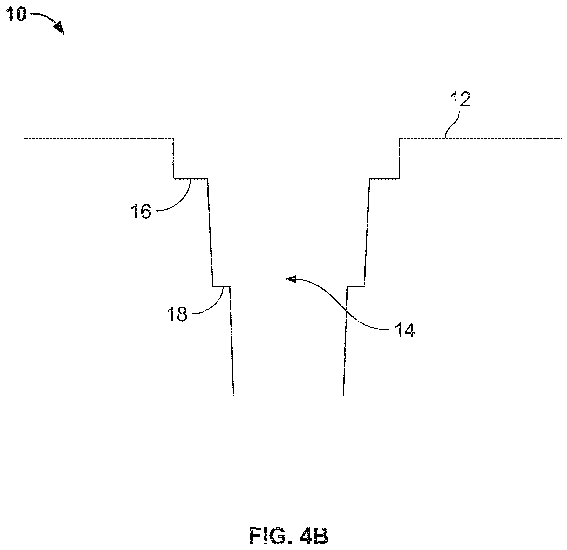

At this point, a selection of an augment type may be made. In some examples, an augment from among augments 100, 200, 300 is chosen. If a collar 110, 210, 310 is to be used with the respective augment 100, 200, 310 implanted into the bone 10, collar trials are used to determine a size of the collar 110, 210, 310. Once the appropriate collar trial is determined, a planar facing reamer is used to further prepare surface 12. The collar trial is then assembled to the cone reamer and stem trial, and any additional components for the trial construct, to assess the joint reconstruction Alternatively, surface 12 of the bone 10 may be further resected using the planar facing reamer to plane the surface of the bone 10 that will receive the collar 110, 210, 310 as shown in FIGS. 4B,5B, and 6B. The planar facing reamer is assembled over the conical reamer component and stem trial and is used to ream the bone 10 until a positive stop is reached and a proximal recessed step 16 is formed within the bone 10.

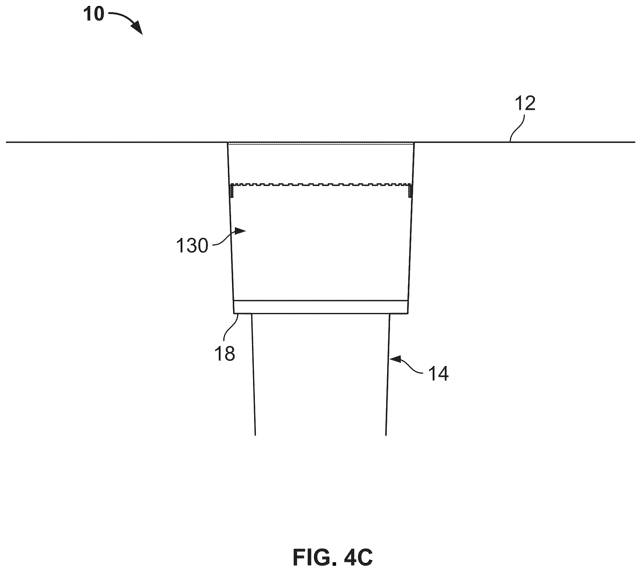

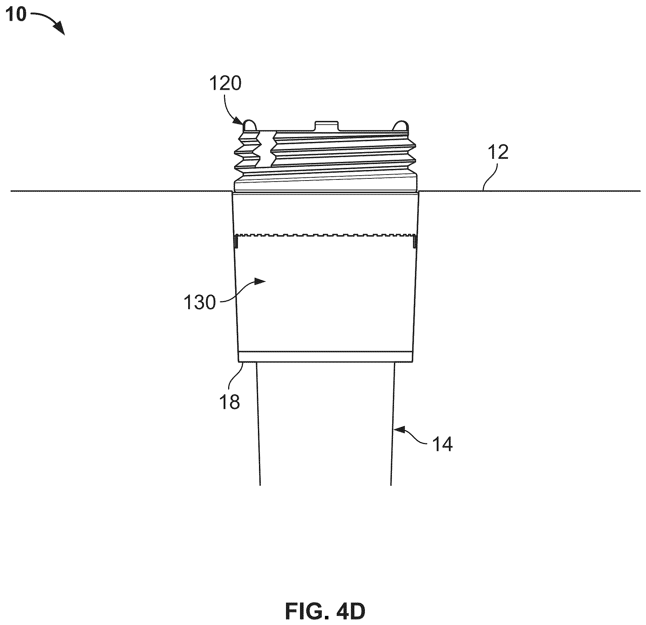

In a sixth step, the selected augment 100, 200, 300 is implanted into the IM canal 14 of the prepared bone 10, as shown in FIGS. 4C, 5C, and 6C. First, a cone or body introducer is used to implant body 130, 230, 330 of the respective augment 100, 200, 300 into the conical cavity of the prepared bone 10 such that that distal recess step 18 is configured for receiving the distal end of the respective augment 100, 200, 300. If augment 100 is being implanted into the prepared bone, the introducer may be used to assemble connector 120 onto body 130 after implantation of body 130, as shown in FIG. 4D. However, in some examples, the connector 120 may be assembled to body 130 prior to implanting body 130 into the resected bone, and the assembled connector 120 and body 130 may be implanted into the conical cavity of the resected bone 10 together. Alternatively, if augment 200, 300 is being implanted into the prepared bone, one can proceed directly to the seventh step below. When implanted, porous portions 115, 135, 215, 235, 315, 335 of the respective augment 100, 200, 300 are positioned to allow for biological fixation of the augment with the prepared bone. Bone cement is susceptible to degradation over time, so biological fixation with components of the augment is desirable to partially alleviate this issue.

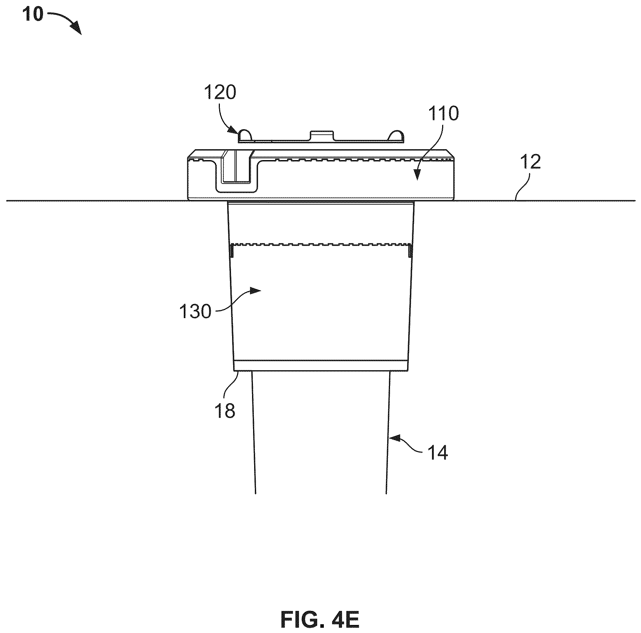

In seventh step, collar 110, 210, 310 is assembled to the respective body 130, 230, 330 implanted into the prepared bone 10, as shown in FIGS. 4E, 5D, and 6D. Again, using the cone or body introducer, collar 110, 210 is assembled to the respective body 130, 230 until collar 110, 210 contacts the planar resected portion or surface 12 of the prepared bone 10. For example, collar 110, 210 may be rotated onto body 130, 230 via the complementary threads on collar 110, 210 and connector 120, 220, respectively. However, if collar 310 is being assembled to body 330, collar 310 is first positioned adjacent to and/or in contact with body 330 and bone surface 12. Once collar 310 is properly positioned, connector 320 is positioned through opening 317 of collar 310, and connector 320 engages collar 310 to body 330 until collar 310 contacts the planar resected portion or surface 12 of the prepared bone 10. For example, connector 320 may be rotated onto collar 310 and body 330 via threads 325 on connector 320 and threads 336 on body 330, respectively. As such, step 329 of connector 320 contacts and/or compresses step 316 of collar 310 to secure collar to body 330 and bone surface 12. One reason this method of assembly is advantageous is because a position of the collar 110, 210, 310 relative to bone surface 12, and a pressure between collar 110, 210, 310 and bone surface 12 when the collar is in contact with bone surface 12, may be adjusted to suit an intended surgical plan. In this manner, the augment may be implanted without excess pressure or insufficient pressure applied to the bone. Further, in this implanted configuration, bone is able to bridge or grow from surface 12 into the respective porous portions 115, 215, 315 of the respective collars 110, 210, 310.

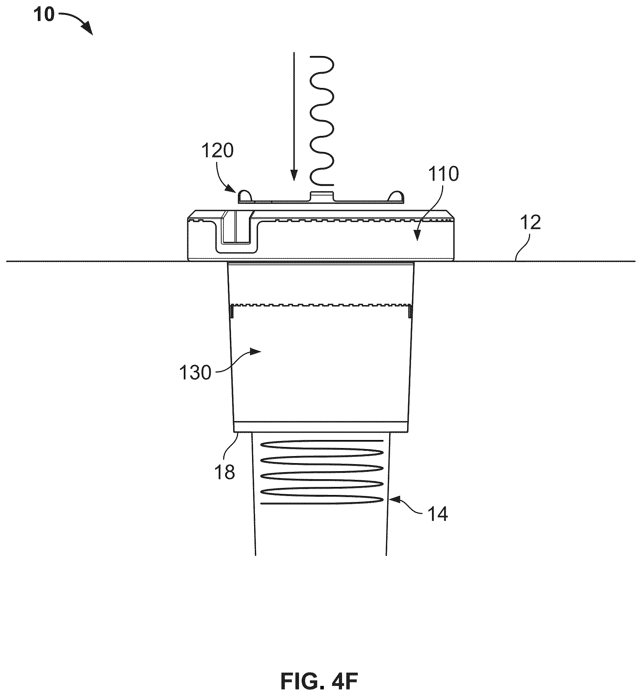

In an eighth step, bone cement is introduced into the implanted augment 100, 200, 300 and prepared portion of the bone 10, as shown in FIG. 4F, 5E, and 6F. Specifically, bone cement is introduced through opening 117, 217, 317 of collar 110, 210, 310 and bone cement continues to flow through the respective augment 100, 200, 300 and into the reamed IM canal 14 of the prepared bone 10. For example, for augment 100, bone cement will flow through opening 127 of connector 120 and then continue to flow through opening 137 of body 130 until reaching the reamed IM canal 14 of the prepared bone 10. For augment 200, bone cement will flow through opening 227 of connector 220 and the continue to flow through opening 237 of body 230 until reaching the reamed IM canal 14 of the prepared bone 10. For augment 300, bone cement will flow through opening 327 of connector 320 and then continue to flow through opening 337 of body 330 until reaching the reamed IM canal 14 of the prepared bone 10.

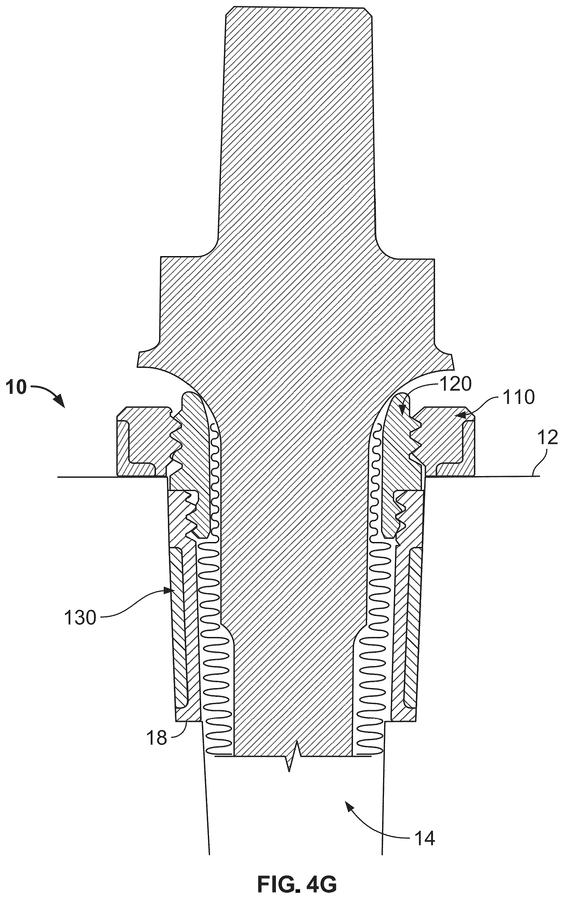

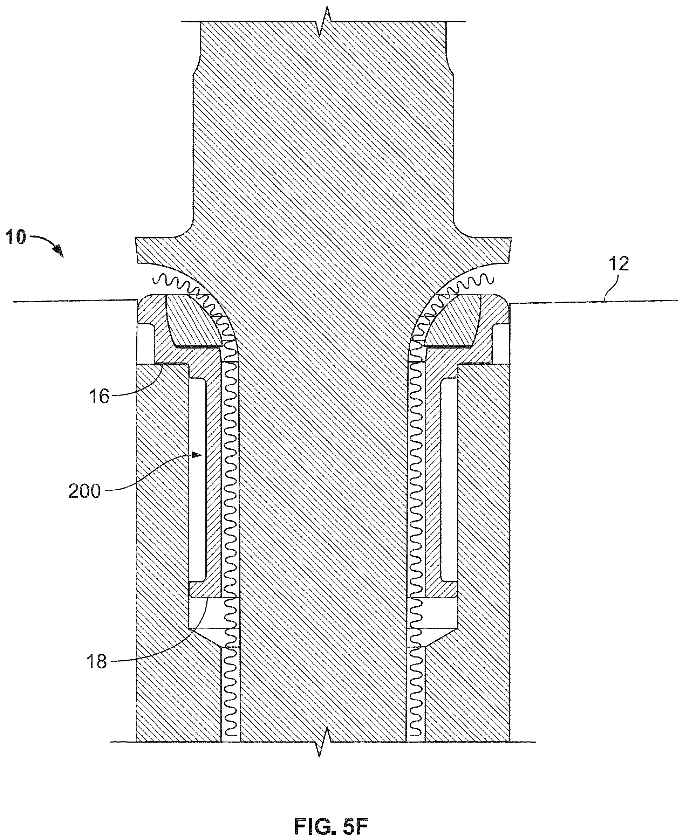

In a ninth step, a stem is introduced through implanted augment 100, 200, 300 and is implanted into the prepared bone 10, as shown in FIG. 4G, 5F, and 6G. The inserted stem is implanted through the openings of the augment 100, 200, 300 and is further anchored to the prepared bone by the previously introduced bone cement. Once implanted, the stem causes the previously introduced bone cement to flow back through the openings of the of the respective augment 100, 200, 300 and out of prepared bone of the patient. Specifically, as the stem is inserted, the bone cement becomes pressurized, which causes the cement to flow back through the respective openings, thus filling any gaps between bone 10 and stem and augment 100, 200, 300 and stem. As such, the method utilizing assembled augment 100, 200, 300 creates a uniform and reproducible cement mantle to support the stem. For example, FIG. 4G illustrates a stem implanted through augment 100 in the prepared bone 10. In another example, FIG. 5F illustrates a stem implanted through augment 200 in the prepared bone. This flow of bone cement ensures that the porous portions 115, 135, 215, 235, 315, 335 of augment 100, 200, 300 are not contacted by the bone cement to ensure that the biological fixation of the respective augment 100, 200, 300 with the prepared bone is not obstructed.

The disclosure herein has been described with reference to particular embodiments, it is to be understood that these embodiments are merely illustrative of the principles and applications of the present disclosure. It is therefore to be understood that numerous modifications may be made to the illustrative embodiments and that other arrangements may be devised without departing from the scope of the present disclosure as defined by the appended claims.

## Claims

1. A modular implant comprising:
a first component configured for receipt within a bone, the first component having a frustoconical shape and a lumen therethrough;
a second component removably engaged to the first component such that a portion of the second component is received within the lumen of the first component; and
a third component removably engaged to the second component such that an inner surface of the third component is engaged to an outer surface of the second component, an outer surface of the third component being configured for engagement with a resected portion of the bone.

2. The modular implant of claim 1, wherein portions of the first component and the third component that interface with the second component are made of materials different from the second component.

3. The modular implant of claims 1 or 2, wherein the second component is made of a polymeric material and at least a portion of the respective first and third components interfacing with the second component is made of a metallic material.

4. The modular implant of any one of claims 1-3, wherein the third component is ring-shaped and has a diameter greater than a maximum diameter of the first component.

5. The modular implant of any one of claims 1-4, wherein the first component includes a first threaded surface, the second component includes a second threaded surface and a third threaded surface and the third component includes a fourth threaded surface, the first threaded surface being engaged with the second threaded surface and the third threaded surface being engaged with the fourth threaded surface.

6. The modular implant of any one of claims 1-5, wherein when the second component is engaged to the first component, the second component extends only partially into the lumen of the first component.

7. The modular implant of any one of claims 1-6, wherein the first component is made of a solid material and a porous material, wherein the porous material is disposed over an outer surface of the solid material, wherein the porous material is configured to enhance biological fixation with the bone.

8. The modular implant of any one of claims 1-7, wherein the third component is made of a solid material and a porous material, wherein the porous material is disposed over an outer surface of the solid material, wherein the porous material is configured to enhance biological fixation with the bone.

9. The modular implant of any one of claims 1-8, wherein a tab extends from a proximal surface of the second component, the tab being configured to engage a distal surface of the third component and partially form a gap between the proximal surface of the second component and the distal surface of the third component.

10. The modular implant of any one of claims 1-9, wherein a notch is formed within an outer surface of the third component, the notch being configured to be engageable by an instrument.

11. The modular implant of any one of claims 1-10, wherein the outer surface of the second component includes a step for engagement to the inner surface of the third component.

12. The modular implant of claim 11, wherein the step forms a gap between a proximal surface of the first component and a distal surface of the third component when the second component is engaged to the first component and the third component is engaged to the second component.

13. A system comprising:
the modular implant of any one of claims 1-12;
a stem disposed through the lumen of the first component and within the bone; and
bone cement configured to fixate the modular implant and stem to the bone.

14. A modular implant comprising:
a first component having a lumen extending therethrough and being configured for receipt within a bone, the first component including a porous portion and a solid portion and having a frustoconical shape; and
a second component removably engaged to the first component such that a portion of the second component is received within the lumen of the first component, the second component comprising a porous portion and solid portion,
wherein the porous portions of the first and second components are configured for engagement with a resected portion of bone, wherein at least a part of the solid portions are configured to engage bone cement.
